# EUROPÄISCHE PATENTSCHRIFT

(11) **EP 0 995 395 B1**
(45) Veröffentlichungstag und Bekanntmachung des Hinweises auf die Patenterteilung: **20.08.2003**
(21) Anmeldenummer: 99120671.5
(22) Anmeldetag: 19.10.1999
(51) Int. Cl.: A61B 5/00

(54) **Diagnosegerät mit Funkverbindung**
Diagnostic instrument comprising radio link
Dispositif de diagnostic ayant des moyens de radiocommunication

(30) Priorität: 23.10.1998 DE 19849123
(43) Veröffentlichungstag der Anmeldung: 26.04.2000
(73) Patentinhaber: Uhlemann, Gisela, 45721 Haltern (DE)
(72) Erfinder: Uhlemann, Gisela, 45721 Haltern (DE)
(74) Vertreter: von Willich, Werner, Dipl.-Phys.

(56) Entgegenhaltungen:
- WO-A-97/25100
- DE-C- 19 707 681

## Beschreibung

Die Erfindung betrifft ein Diagnosegerät, insbesondere Herzdiagnosegerät, mit einem Gehäuse, einer im Gehäuse angeordneten Spannungsversorgung, im Gehäuse angeordneter Sende- und Empfängerelektronik und am Gehäuse angeordneten Elektroden zur Anlage an den Körper und Messung durch Aufnahme von elektrischen Körpersignalen.

Ein Herzdiagnosegerät der angegebenen Gattung ist aus der DE 197 07 681 C1 bekannt. Bei dem bekannten Gerät werden mit Hilfe von auf der Rückseite des Gerätes (fest) angebrachten Elektroden elektrische Körpersignale, zum Beispiel EKG-Signale aufgenommen und über ein in dem selben Gehäuse angebrachtes Funkfernmeldegerät, zum Beispiel ein Mobilfunk-Telefon, an eine entfernte Stelle weitergeleitet. Diese entfernte Stelle kann zum Beispiel ein Krankenhaus, eine Notrufstation oder dergleichen sein.

Es ist aber ersichtlich, daß die Funksignale, vor allem diejenigen, die das Gerät selbst aussendet, die Patienten, die einen implantierten Herzschrittmacher tragen, lebensbedrohlich gefährden können. Zum Beispiel dürfen bekanntermaßen Funktelefone nicht in Flugzeugen oder überhaupt in der Nähe empfindlicher Elektronik eingesetzt werden.

Aufgabe der Erfindung ist es daher, ein gattungsgemäßes Diagnosegerät so weiterzubilden, daß eine Gefährdung des Patienten möglichst vermieden wird.

Dies wird erfindungsgemäß dadurch erreicht, daß bei einer Betätigung des Gerätes für die Aufnahme von elektrischen Körpersignalen zumindest die Sendefunktion und ggfs. weitere nicht der Messung dienende Funktionen abgeschaltet werden.

Die erfindungsgemäße Lösung hat insbesondere folgende Vorteile:

Derartige Diagnosegeräte werden insbesondere bei Patienten eingesetzt, die schon herzgeschädigt sind. Solche Patienten tragen aber häufig einen implantierten Herzschrittmacher (weltweit ca. 1 Million), was oft, insbesondere in Notfällen, übersehen wird. Die Patienten weisen möglicherweise im entscheidenden Moment nicht darauf hin oder sind aufgrund ihres Zustandes nicht dazu in der Lage. Gerade Herzschrittmacher werden aber durch die - bei der Anwendung des Diagnosegerätes auch dem Herzen besonders nahe - Sende-und Empfängerelektronik des Funktelefons erheblich gestört und es können gefährliche Herzrhythmusstörungen ausgelöst werden. Dies wird dadurch vermieden, daß, wenn das Gerät in einen zur Messung einsatzfertigen Zustand gebracht wird, erfindungsgemäß die Sende-/Empfängereinrichtungen automatisch ausgeschaltet werden.

Dabei können die Schritte z.B. das Umschalten des Anzeigefeldes auf Messung, die Betätigung einer Meßtaste oder dergleichen sein.

Besonders bevorzugt liegen die Elektroden (i.d.R. 3 bis 5 Elektroden) in einem ersten Zustand des Diagnosegerätes nicht frei (d.h. sie sind zum Beispiel abgedeckt) und werden durch das Freilegen mindestens einer Elektrode zumindest die Sendefunktion und ggfs. weitere nicht der Messung dienende Funktionen abgeschaltet.

Besonders bevorzugt sind die Elektroden durch eine Gesamt- oder Einzelabdeckung abgedeckt, deren Entfernung zumindest die Sendefunktion und ggfs. weitere nicht der Messung dienende Funktionen abschaltet.

Eine solche Abdeckung macht ohne weiteres sinnfällig, daß zum Benutzen des Gerätes als Diagnosegerät eine Handlung vorgenommen werden muß. Diese Handlung schaltet dann (etwa durch mit dem Deckel bzw. den Abdeckungen verbundene Mikroschalter) die Fernmeldefunktion des Gerätes ab, wobei im Falle mehrerer Abdeckungen die Entfernung einer Abdeckung ausreicht, um die Abschaltung zu bewirken.

Ebenfalls wird das Anstecken eines Patientenkabels als eine entsprechende Betätigung des Gerätes zur Messung verstanden.

Nachfolgend wird die Erfindung anhand bevorzugter Ausführungsbeispiele und unter Bezugnahme auf die beigefügten Zeichnungen, auf die wegen ihrer großen Klarheit und Übersichtlichkeit hinsichtlich der Offenbarung ausdrücklich verwiesen wir, noch näher erläutert.

Es zeigen:
- Fig. 1:: Die Rückseite eines erfindungsgemäßen Diagnosegerätes mit drei Elektroden und deren öffenbaren Abdeckungen, in geöffnetem Zustand;
- Fig. 2:: Die Vorderseite des erfindungsgemäßen Gerätes im Meßzustand mit den zugehörigen Bedienungstasten und dem zugehörigen Anzeigefeld sowie eine lange Schmalseite mit den zugehörigen Bedienungstasten, die als Folientasten ausgebildet sind;
- Fig. 3:: Die Vorderseite des erfindungsgemäßen Diagnosegerätes im Funkzustand mit der Anzeige einer normalen Tastatur eines Funktelefons; und
- Fig. 4:: Das Anzeigefeld des Diagnosegerätes im Zustand zur Überprüfung der Batteriekapazität eines Herzschrittmachers.

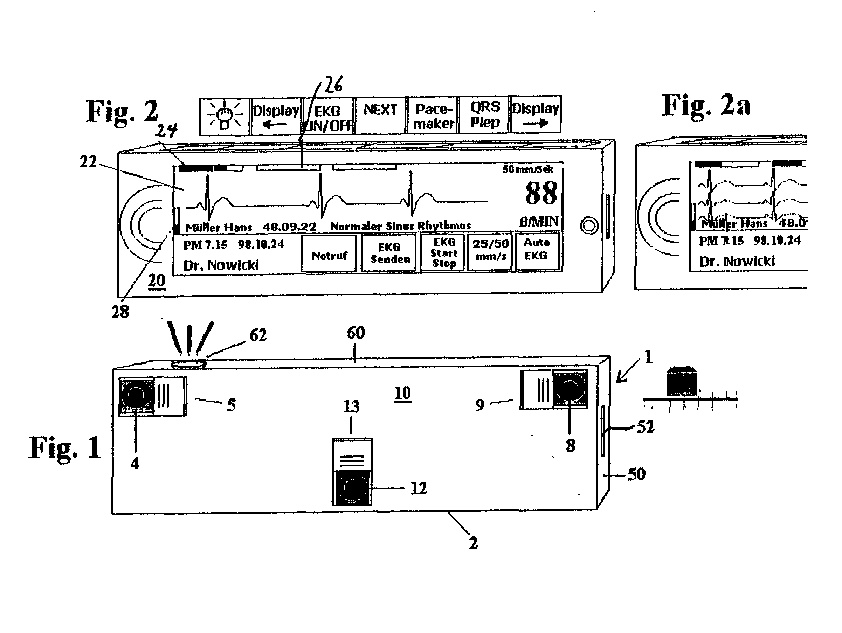

In Fig. 1 ist die eine Flachseite 10 (Rückseite) des Gehäuses 2 des insgesamt mit 1 bezeichneten Diagnosegerätes gezeigt. Das Diagnosegerät wird vorzugsweise als Herzdiagnosegerät zur Messung der elektrischen Oberflächensignale für die Bestimmung der Herzfunktion eingesetzt, kann aber auch, wie an sich bekannt, vgl. DE-C-36 36 996, für die Messung von Gehimströmen genutzt werden. Dazu ist jedoch erforderlich, eine etwa tausendfache Verstärkung der gemessenen elektrischen Signale in Vergleich zum beschriebenen Herzdiagnosegerät durchzuführen. Auch dort gerade ist es also wesentlich, daß keine Störungen durch Fremdsignale auftreten. Auf der Rückseite 10 sind in möglichst großem Abstand Elektroden, im Ausführungsbeispiel drei Elektroden, 4, 8 und 12, angeordnet. Die Elektroden können aber auch abnehmbar sein, um einen größeren Abstand zwischen den Meßpunkten erzielen zu können, vergleiche DE 196 02 347 A1 Jede der EKG-Aufnahme-Elektroden 4, 8 und 12 ist durch eine zugehörige Abdeckung, im vorliegenden Falle durch einen an einer Seite angelenkten Schiebedeckel 5 bzw. 9 bzw. 13 abgedeckt. Es können aber auch bspw. Klappdeckel verwendet werden.

Das Öffnen der Schiebedeckel 5 bzw. 9 bzw. 13 schaltet je einzeln die Funkfunktion des Gerätes 1 ab, allgemeiner alle nicht zur Messung erforderlichen Funktionen, die z.B. einen Herzschrittmacher stören könnten. Zusätzlich wird bevorzugt die EKG-Elektrode mit einer Feder nach oben gedrückt und kann dort vorzugsweise arretiert werden, so daß sie leichter an den Körper anlegbar ist.

Fig. 2 zeigt die der Seite 10 gegenüberliegende Flachseite 20 des Gerätes 1, das im vorliegenden Falle nicht nur als allgemeines Funktelefon, sondern auch als Spezialausführung für einen Arzt, sozusagen als "Arzt-EKG-Handy" ausgebildet ist.

Das Anzeigefeld 22 zeigt beispielsweise die - z.B. durch eine Patientenkarte oder auf sonstige Weise eingegebenen - Patientendaten (Name, Geburtsdatum) sowie die Grafik des EKG, das als Ein -oder Dreikanal-Aufzeichnung (letztere in der Fig. 2a dargestellt) aufgenommen und dargestellt werden kann. Ferner können von vornherein der Name des behandelnden Arztes und Datum sowie Uhrzeit angegeben sein, wie im bevorzugten Ausführungsbeispiel gezeigt. Die Herzfrequenz wird in Ziffern (hier als Beispiel; "88") angezeigt. Bei 28 ist die 1 mV-Eichung angezeigt.

Das Gerät trägt auf seiner Flachseite 20 auch eine, hier innerhalb des Anzeigefeldes in der EKG-Funktion angezeigte, an sich bekannte, "Notruf"-Taste sowie eine "EKG/senden"-Taste, die aber erfindungsgemäß erst nach Bedeckung der Elektroden funktionieren. Es kann gegebenenfalls aber auch vorgesehen sein, daß z.B. die Notruftaste auch bei freiliegenden Elektroden eine Rufaussendung veranlaßt.

Mit der Taste "EKG/senden" wird das EKG im EKG-Speicher 24 gesendet, z.B. in die Praxis des behandelnden Arztes. Die Taste "EKG/Start/Stop" lößt die Messung aus, mit der Taste "25/50 mm/s" läßt sich die Schreibgeschwindigkeit umschalten. Die Schreibgeschwindigkeit kann, wie auf dem Anzeigefeld 22 rechts oben dargestellt, ebenfalls angezeigt werden.

Die Taste "Auto EKG" wird benutzt, wenn z.B. eine EKG-Aufzeichnung mit verschiedenen Ableitungen vorgenommen werden soll. Dazu wird ein Patientenkabel (nicht gezeigt) in die Steckbuchse 52 an der kurzen Schmalseite 50 des Gehäuses 2 eingesteckt. Bei Anwendung eines Patientenkabels wird die Funkfunktion automatisch ausgeschaltet, sobald das Patientenkabel in das Gerät gesteckt (52) wird. Mit dem Einsatz eines Patientenkabels kann automatisch das übliche EKG (nach Einthoven I, II, III, Goldberg aVR, aVL, aVF und Wilson V1-6) aufgezeichnet und im Gerät gespeichert werden.Das Einstecken des Patientenkabels hat die gleiche Wirkung wie das Öffnen der Schiebedeckel 5 bzw. 9 bzw. 13, d.h. die Funkelektronik des Gerätes 1 wird abgeschaltet.

Fig. 2 zeigt auch einen Ausschnitt einer Aufsicht auf die lange Schmalseite 30 des Gehäuses 2 des Gerätes 1. Die Taste "Next" gibt den nächsten Speicher (z.B. 26 nach 24) frei, um es zu ermöglichen, EKGs des gleichen Patienten oder EKGs unterschiedlicher Patienten getrennt aufzuzeichnen. Die Tasten "Display/<---" und "Display/--->" erlauben die gespeicherten EKG-Daten nachträglich anzusehen.

Die mit einem Lampensymbol gekennzeichnete Taste ermöglicht die Einschaltung einer bevorzugt eingebauten Diagnoselampe 62, die auf der weiteren langen Schmalseite 60 angeordnet ist und die z.B. zur Überprüfung der Pupillenreaktion dienen kann.

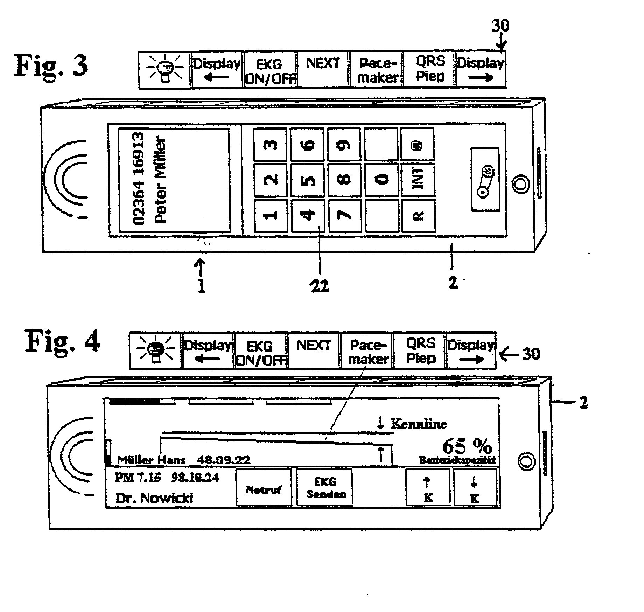

Das Anzeigefeld 22 ist durch die Taste "EKG/ON/OFF" umschaltbar und kann im Falle der AUS-Schaltung der EKG-Funktion die Tastatur eines Funktelefons darstellen, s. Fig. 3, die, wie die oben erläuterten virtuellen Tasten im Meßbetrieb, mit Stift oder Finger durch Druck bedienbar ist. Die Bedienung des Gesamtgerätes kann aber selbstverständlich auch für eine Sprachsteuerung ausgelegt sein.

Bevorzugt weisen die EKG- Elektroden einen Fühlmechanismus auf, der in der Lage ist, das Vorhandensein oder Fehlen eines (Haut-)Kontaktes festzustellen. Dann kann auch eine automatische Speicherung der Aufzeichnung bewirkt werden, sobald der Hautkontakt unterbrochen ist.

Auf die Elektroden sind auch Klebeelektroden aufklippbar.

Vorzugsweise ist das Gerät auch zum Empfang von Nachrichten, die schriftlich auf dem Anzeigefeld dargestellt werden, eingerichtet.

Das Gerät ist in der Ausführungsform der Fig. 4 mit einer Zusatzfunktion versehen, die zum Messen der Batteriekapazität eines Herzschrittmachers eingerichtet ist. Dazu wird das Impulsplateau des Schrittmachers verbreitert dargestellt und mit einer Kennlinie verglichen.

Eine induktive Abfrage der Herzschrittmacherdaten ist ebenfalls vorgesehen.

Entsprechende Funktionen können durch die entsprechenden Tasten, die dann auf dem Anzeigefeld 22 dargestellt werden, oder durch die Doppelbelegung von Tasten ausgelöst werden, vgl. auch die Darstellung der Schmalseite 30 in Fig. 4. Die durch Pfeile im Anzeigefeld 22 angedeutete Differenz zwischen der Kennlinie und der Kurve des Herzschrittmachers gibt Auskunft über die Batteriekapazität.

## Patentansprüche

1. Diagnosegerät, insbesondere Herzdiagnosegerät, mit
einem Gehäuse (2),
einer im Gehäuse (2) angeordneten Spannungsversorgung,
im Gehäuse (2) angeordneter Sende- und Empfängerelektronik und am Gehäuse (2) angeordneten Elektroden (4, 8, 12) zur Anlage an den Körper und Messung durch Aufnahme von elektrischen Körpersignalen,
**dadurch gekennzeichnet, daß**
bei einer Betätigung des Gerätes für die Aufnahme von elektrischen Körpersignalen zumindest die Sendefunktion und ggfs. weitere nicht der Messung dienende Funktionen abgeschaltet werden.

2. Diagnosegerät nach Anspruch 1
**dadurch gekennzeichnet, daß**
die Elektroden (4. 8, 12) in einem ersten Zustand des Diagnosegerätes nicht freiliegen und
durch das Freilegen (5, 9, 13) mindestens einer Elektrode (4, 8, 12) zumindest die Sendefunktion und ggfs. weitere nicht der Messung dienende Funktionen abgeschaltet werden.

3. Diagnosegerät nach Anspruch 1 oder 2,
**dadurch gekennzeichnet, daß**
die Elektroden durch eine Gesamt- oder Einzelabdeckung abgedeckt sind, deren Entfernung zumindest die Sendefunktion und ggfs. weitere nicht der Messung dienende Funktionen abschaltet.

## Claims

1. A diagnostic apparatus, particularly a cardiac diagnostic apparatus, with
a housing (2),
a power supply unit provided in the housing (2),
transmit-receive electronics provided in the housing (2),
and electrodes (4, 8, 12) provided on the housing (2), for application to the body and measurement operations by recording electrical body signals,
**characterized in that**
when activating the apparatus for recording electrical body signals, at least the transmit function and, where appropriate, other functions not serving for measurement are disconnected.

2. A diagnostic apparatus according to claim 1,
**characterized in that**
the electrodes (4, 8, 12) in a first condition of the diagnostic apparatus are not uncovered, and,
by uncovering (5, 9, 13) at least one electrode (4, 8, 12) at least the transmit function and, where appropriate, other functions not serving for measurement are disconnected.

3. A diagnostic apparatus according to claim 1 or 2,
**characterized in that**
the electrodes are covered by an overall or individual cover, the removal of which at least disconnects the transmit function and, where appropriate, other functions not serving for measurement.

## Revendications

1. Dispositif de diagnostic, en particulier dispositif de diagnostic cardiaque, avec
un boîtier (2),
une source de tension disposée dans le boîtier (2),
une électronique d'émission et de réception disposée dans le boîtier (2),
et des électrodes (4, 8, 12) disposées sur le boîtier (2) destinées à l'application sur le corps et à la mesure par capture de signaux électriques corporels,
**caractérisé en ce que**
lors d'un actionnement du dispositif pour capter des signaux électriques corporels, au moins la fonction d'émission et le cas échéant d'autres fonctions ne servant pas à la mesure sont déconnectées.

2. Dispositif de diagnostic selon la revendication 1,
**caractérisé en ce que**
les électrodes (4, 8, 12) ne sont pas disposées librement dans un premier état du dispositif de diagnostic et,
par le dégagement (5, 9, 13) d'au moins une électrode (4, 8, 12), au moins la fonction d'émission et le cas échéant d'autres fonctions ne servant pas à la mesure sont déconnectées.

3. Dispositif de diagnostic selon la revendication 1 ou 2,
**caractérisé en ce que**
les électrodes sont couvertes par un couvercle général ou individuel, dont l'enlèvement déconnecte au moins la fonction d'émission et le cas échéant d'autres fonctions ne servant pas à la mesure.
